# EUROPEAN PATENT APPLICATION

(11) **EP 4 810 072 A1**
(43) Date of publication of application: **23.09.2026**
(21) Application number: 25164422.5
(22) Date of filing: 18.03.2025
(51) Int. Cl.: A61M 1/00, A61M 3/02

(54) **A TIP ASSEMBLY FOR A PULSE LAVAGE DEVICE**

(71) Applicant: Mölnlycke Health Care AB, 431 21 Mölndal (SE)
(72) Inventor: GUNGNER, Mattias, 441 60 Alingsås (SE)
(74) Representative: Kransell & Wennborg KB

(57) **Abstract**

The present disclosure generally relates to a tip assembly (1) for a pulse lavage device (2), wherein said tip assembly (1) comprises:
- a connector portion (3) for attaching the tip assembly (1) to the pulse lavage device (2);
- an inner irrigation tube (4) extending from the connector portion (3), wherein the irrigation tube (4) has a length, L₁; and
- an outer suction tube (5) configured to be detachably attached to the connector portion (3) and to encircle at least a portion of the length, L₁, such that a suction channel (6) is defined between the irrigation tube (4) and the outer suction tube (5) in a mounted state.

The present disclosure also relates to a pulse lavage device (2) comprising the tip assembly (1).

## Description

### TECHNICAL FIELD

The present disclosure generally relates to a tip assembly for a pulse lavage device, wherein the tip assembly comprises a connector portion for attaching the tip assembly to the pulse lavage device, an inner irrigation tube extending from the connector portion, and an outer suction tube encircling at least a portion of the irrigation tube. The present disclosure also relates to a pulse lavage device comprising the tip assembly.

### BACKGROUND

Pulse lavage devices are commonly used in orthopedic procedures to irrigate and clean a surgical site and prepare a bone bed for an implant. Removing bone fragments, blood, and debris is important to secure proper fixation of the prosthesis or implant. Effective cleaning of the bone surface also improves cement fixation, which yields a better implant stability.

A pulse lavage device typically comprises a handpiece connected to a source of pressurized irrigation fluid and a suction system to remove the fluid and debris from the surgical site.

While pulse lavage devices are widely used in various surgical procedures, such devices are associated with various challenges in clinical practice. For example, debris and smaller bone fragments may become stuck in the suction tube of the pulse lavage device, which may lead to blockages. Such blockages may reduce the suction efficiency and may disrupt the cleaning process. To avoid this, the surgical staff often relies on a separate suction device instead of using the built-in suction function of the pulse lavage system.

The need to alternate between irrigation and suction using different devices is associated with significantly increased procedural complexity.

Other procedures, which also require alternation between separate irrigation and suction systems are procedures involving joint replacement or fracture fixation of smaller joints, e.g. the elbow joint, or small bones, such as the ulna and radius.

Current pulse lavage devices are generally designed for larger bone cavities and are not suited for cleaning inside smaller bones, such as during the preparation of the intramedullary canal before implant placement.

To date, such small bone cavities are typically irrigated using separate syringes to flush the canal with irrigation fluid. However, the need to manually switch between syringes for irrigation and separate suction tools (e.g. the pulse lavage device) for fluid removal is generally inconvenient and a cumbersome procedure for the surgical staff. Furthermore, a syringe has a limited debriding capacity, as it lacks the pressure and flow control of a pulse lavage device.

In view of the challenges mentioned above, there is a need for an improved pulse lavage device that facilitates irrigation and suction, reduces procedural complexity and which offers a better adaptability for different surgical scenarios.

### SUMMARY

In view of the above-mentioned challenges, it is an object of the present disclosure to provide improvements in the field of pulse lavage devices, particularly to facilitate both irrigation and suction within bone cavities while reducing procedural complexity and ensuring ease of use for medical personnel in various surgical scenarios.

According to a first aspect, there is provided a tip assembly for a pulse lavage device, the tip assembly comprising:
- a connector portion for attaching the tip assembly to the pulse lavage device;
- an inner irrigation tube extending from the connector portion, wherein the irrigation tube has a length, L₁; and
- an outer suction tube configured to be detachably attached to the connector portion and to encircle at least a portion of the length, L₁ of the irrigation tube such that a suction channel is defined between the irrigation tube and the outer suction tube in a mounted state.

The present disclosure is based on the realization that a tip assembly as described hereinabove is associated with a significantly improved handling and procedural efficiency for the surgical staff in medical scenarios requiring irrigation and suction.

The detachable outer suction tube allows the surgical staff to quickly adapt to different surgical scenarios. For example, when focused irrigation is required in confined spaces, such as small bone cavities, the suction tube may be removed, and the irrigation tube may be used (alone) for precise fluid delivery without obstruction. Conversely, when both irrigation and suction are needed for efficient fluid and debris removal, the suction tube can be reattached to restore full suction capability.

In cases where small bone fragments and debris become stuck in the suction tube, the suction tube may easily be detached, cleaned, and re-attached again.

Furthermore, in scenarios where the medical personnel prefer to use a separate suction tool instead of the built-in suction function of the pulse lavage device, the removable suction tube allows for this possibility as well.

The connector portion of the tip assembly serves as the interface between the pulse lavage device and the irrigation and suction tubes. The connector portion allows for fluid connection between the pulse lavage device and the inner irrigation tube, as well as the outer suction tube. The connector portion may comprise internal conduits which secure that irrigation fluid is directed into the irrigation tube, and that suction forces are transmitted to the suction tube, respectively.

In a mounted state, the irrigation tube is arranged within the suction tube such that a suction channel is defined between the irrigation tube and the outer suction tube. The suction channel allows for efficient removal of irrigation fluid, bone fragments, and debris.

In exemplary embodiments, the suction tube may be configured to encircle at least 80%, preferably from 80 to 98% of the length, L₁, of the irrigation tube in the mounted state.

Accordingly, a large proportion of the irrigation tube is arranged within the suction tube in the mounted state. In some embodiments, the outer suction tube may be arranged to encircle the entire length, L₁, of the irrigation tube in the mounted state. However, in other embodiments, it may be beneficial that the irrigation tube extends slightly beyond the suction tube to facilitate irrigation. The distal end of the irrigation tube typically comprises openings for irrigation of fluid. The fact that the irrigation tube extends beyond the suction tube secures that irrigation is not obstructed by the suction tube during use.

In exemplary embodiments, the irrigation tube has an outer surface and an inner surface, the suction tube having an outer surface and an inner surface, wherein the maximum distance, A₁, between the outer surface of the irrigation tube and the inner surface of the suction tube is from 2 to 10 mm, preferably from 2 to 6 mm in the mounted state.

A distance in this range secures that the suction channel defined between the irrigation tube and the suction tube is large enough to remove fluid, debris and smaller bone fragments from the surgical site. If the distance, A₁, is too large, the tip assembly may become too large and bulky, making it difficult to access bone cavities or bone channels. In contrast, if the suction channel is too small, debris and bone fragments may accumulate in the suction tube, which may enhance the risk of clogging and impair the suction efficiency.

A distance in the above-mentioned range secures that the suction is strong enough to efficiently remove irrigation fluid and debris, and that that the suction channel remains open and unobstructed during use.

The irrigation tube may be arranged centrally in the suction tube. Alternatively, it may be arranged in an offset position with respect to the suction tube.

The central arrangement may be beneficial from a manufacturing perspective. The offset arrangement may be beneficial to create a larger suction channel, while still maintaining a compact overall design of the tip assembly. A too large tip assembly may prevent proper irrigation and suction in confined surgical sites.

Accordingly, in exemplary embodiments, the irrigation tube may be arranged in an off-center position within the suction tube such that a central axis of the irrigation tube is offset from a central axis of the suction tube in the mounted state.

In exemplary embodiments, the irrigation tube has a diameter, D₁, of from 1.5 to 7 mm, preferably from 3 to 5 mm.

An irrigation tube having a diameter in this range allows for precise access to confined anatomical spaces, such as the intramedullary canals of small bones (e.g., ulna and radius). These spaces are often difficult to clean with conventional pulse lavage tips. As mentioned hereinbefore, the surgical staff must typically use separate syringes to access such small canals and cavities.

A diameter in the above-mentioned range is beneficial to maintain an optimal pressure and flow rate such that the debriding efficiency is improved (compared to manual syringe irrigation). Furthermore, a diameter in this range improves the precision and allows for an improved control when irrigating complex or narrow anatomical structures.

In exemplary embodiments, the suction tube may have a diameter, D₂, of from 3.5 to 15 mm, preferably from 8 to 11 mm.

A suction tube with a diameter in this range is beneficial to secure efficient fluid and debris removal. Accumulation of debris and small bone fragments during suction are prevented, or at least significantly reduced. Furthermore, a suction tube having a diameter in the above-mentioned range ensures an overall compact and convenient design of the tip assembly.

In exemplary embodiments, the irrigation tube may comprise a bendable polymeric material.

The irrigation tube is typically formed from a flexible and bendable polymeric material. This way, the irrigation tube may be inserted into confined anatomical spaces, such as bone cavities and intramedullary canals, and bend or flex, as needed.

Typically, the suction tube is stiffer than the irrigation tube. The suction tube preferably has a higher stiffness to secure that it remains stable and provides effective fluid removal without collapsing or deforming during use.

**In** exemplary embodiments, the bendable polymeric material is selected from the group consisting of polyurethane, polycarbonate, polyethylene, and/or silicone.

An irrigation tube comprising or being formed by such a material is biocompatible, flexible, durable and safe to use.

The suction tube may be detachably attached to the connector portion by any conceivable means and is not limited to a specific attachment mechanism.

In exemplary embodiments, the suction tube may be detachably attached to the connector portion via a bayonet coupling, snap-fit mechanism, or threaded coupling,

These detachable attachment mechanisms facilitate cleaning and replacement or the suction tube and also improve the flexibility for the surgical staff.

In exemplary embodiments, the irrigation tube has a proximal end and a distal end; the suction tube having a proximal end and a distal end, wherein the proximal ends of the irrigation tube and the suction tube are attached to the connector portion in the mounted state; the distal end of the irrigation tube comprising one or more openings, wherein the one or more openings may be arranged in a direction radial to a central axis of the irrigation tube.

The radial arrangement of the opening(s) secures a uniform lateral fluid distribution around the tip of the irrigation tube such that efficient cleaning of bone surfaces and cavities can be accomplished.

The one or more openings may be provided in the irrigation tube or in a separate tip attached to the irrigation tube.

Accordingly, in exemplary embodiments, the distal end of the irrigation tube may comprise a separate tip attached to the distal end, and wherein the separate tip comprises the one or more openings.

This may be beneficial from a manufacturing perspective.

In exemplary embodiments, the separate tip comprises a plurality of openings, wherein the openings may be circumferentially spaced around the separate tip of the irrigation tube relative to the central axis of the irrigation tube.

This configuration improves the irrigation efficiency since fluid is directed outwardly in multiple directions. Accordingly, an improved coverage at the surgical site is provided, which is beneficial to properly debride and clean bone surfaces and cavities.

It is also conceivable, in embodiments where a separate tip is not utilized, that the distal end of the irrigation tube comprises a plurality of openings, wherein the openings are circumferentially spaced around the distal end of the irrigation tube relative to the central axis of the irrigation tube.

In exemplary embodiments, the distal end of the irrigation tube may extend beyond the distal end of the suction tube.

This configuration is beneficial to secure unobstructed fluid delivery during irrigation, i.e. preventing interference from the suction tube.

In exemplary embodiments, the connector portion may comprise an annular element having an interior surface and an exterior surface, wherein the interior surface or the exterior surface is threaded; the proximal end of the suction tube comprising threads configured to engage with the threaded interior or exterior surface of the annular element.

This configuration secures that the suction tube can be easily attached and detached, i.e. it allow for a quick assembly and de-assembly. This is beneficial whenever cleaning, replacement, or reconfiguration of the suction tube is needed during a surgical procedure.

Furthermore, the threaded connection is generally stable and secure, i.e. it maintains a sealed interface, which is beneficial to prevent fluid leakage and accidental disconnection during use.

According to another aspect, there is provided a pulse lavage device comprising the tip assembly described hereinbefore.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF DRAWINGS

The various aspects of the present disclosure, including its particular features and advantages, will be readily understood from the following detailed description and the accompanying drawings, in which:
Figure 1 schematically illustrates a pulse lavage device comprising the tip assembly according to an exemplary embodiment of the present disclosure.
Figure 2A schematically illustrates a tip assembly according to an exemplary embodiment of the present disclosure, in which the suction tube has been detached.
Figure 2B illustrates a cross-sectional view of the irrigation tube of the tip assembly in figure 2A along the line A-A.
Figure 2C schematically illustrates a tip assembly according to an exemplary embodiment of the present disclosure, illustrating that the suction tube is detached from the irrigation tube.
Figure 3B illustrates the connector portion of the tip assembly and how the suction tube is attached to the connector portion according to an exemplary embodiment of the present disclosure.
Figure 4A illustrates a tip assembly according to an exemplary embodiment of the present disclosure, wherein the suction tube is attached to the connector portion.
Figure 4B illustrates a cross-sectional view of the tip assembly in figure 2A along the line B-B, in which the irrigation tube is disposed centrally within the suction tube.
Figure 5 illustrates a cross-sectional view of the tip assembly in figure 2A along the line B-B according to an alternative embodiment of the present disclosure, in which the irrigation tube is disposed in an off-set position within the suction tube.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like references refer to like elements throughout.

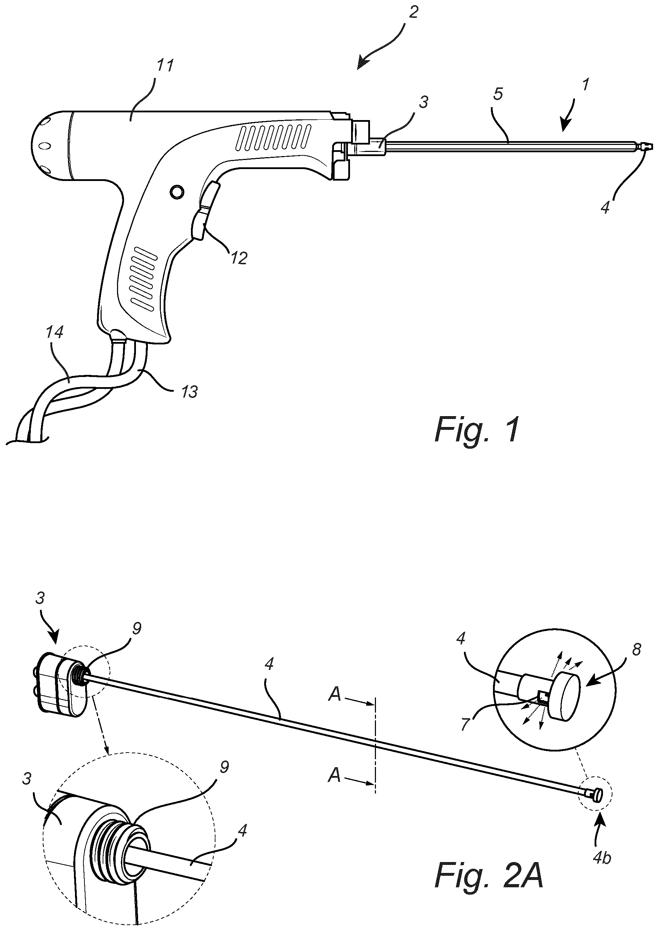

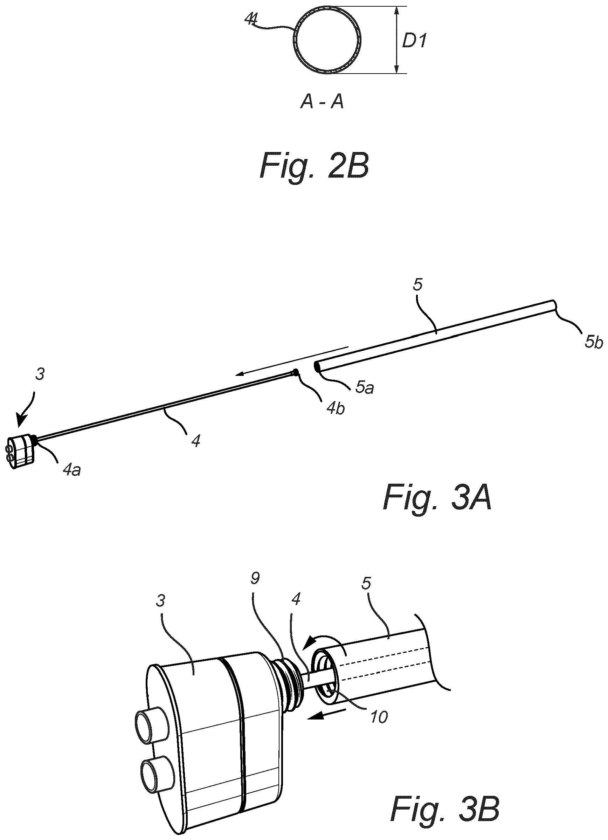

A pulse lavage device is schematically illustrated in figure 1, and the various aspects of the tip assembly for use with the pulse lavage device are illustrated in figures 2A, 2B, 3A, 3B, 4A, 4B, and 5.

The tip assembly 1 comprises
- a connector portion 3 for attaching the tip assembly 1 to the pulse lavage device 2;
- an inner irrigation tube 4 extending from the connector portion 3, wherein the irrigation tube 4 has a length, L₁; and
- an outer suction tube 5 configured to be detachably attached to the connector portion 3 and to encircle at least a portion of the length, L₁, of the irrigation tube 4 such that a suction channel 6 is defined between the irrigation tube 4 and the outer suction tube 5 in a mounted state.

As used herein, the term "pulse lavage device" means a surgical irrigation and suction device configured to deliver irrigation fluid to a surgical site and to remove irrigation fluid, debris and small bone fragments from the surgical site. Fluid and debris may be drawn from the surgical site by means of a suction system, which may operate with a negative pressure gradient.

As illustrated in figure 1, the pulse lavage device 2 may comprise a handpiece 11, which serves as the main body of the device 2. The handpiece 11 may have the general shape of a gun. The handpiece 11 houses the internal mechanisms for generating pulsed irrigation flow and for regulating suction flow.

The pulse lavage device 2 typically comprises a trigger element 12. The trigger element 12 may e.g. be formed as button that can be pressed for activation. When pressed, pulsed delivery of irrigation fluid is accomplished. The trigger element 12 may also regulate suction flow.

The pulse lavage device 2 comprises at least one fluid conduit and at least one separate suction conduit (not shown). The irrigation tube 4 of the tubing assembly 1 may be connected to an irrigation source (e.g. a saline bag) by means of the fluid conduit in the device. The irrigation source may be arranged in the hospital room and may be connected to the pulse lavage device 2 by means of a fluid tubing 13.

The suction tube 5 of the tubing assembly 1 may be connected to suction system by means of the suction conduit. The suction system may operate by creating negative pressure to draw waste fluid and debris away from the surgical site. The suction system may e.g. be an external vacuum source, such as a hospital suction system or a portable vacuum pump. When suction is activated, the pressure inside the suction conduit and suction tube 5 drops below atmospheric pressure. The pressure differential between the surgical site and the suction tube 6 causes fluid and debris to be drawn into the suction tube 5.

A suction tubing 14 is typically used to connect the pulse lavage device 2 to a separate suction system.

The pulse lavage device 2 may be used in orthopedic procedures to prepare bone surfaces for fracture treatment or implant fixation. The pulse lavage device 2 is beneficial to clean bone fragments and remove necrotic tissue. Proper debridement enhances bone healing and reduces post-operative complications. In e.g. joint replacement surgeries (e.g., hip, knee, and elbow arthroplasty), the pulse lavage device 2 may be used to prepare the bone bed before placement of a prosthesis.

As used herein, the term "tip assembly" means a removable component or assembly that is to be connected to a pulse lavage device. The tip assembly facilitates the delivery of irrigation fluid as well as removal of fluid and debris from the surgical site. The tip assembly comprises a connector portion for attaching the tip assembly to the pulse lavage device, an inner irrigation tube for directing irrigation fluid to the surgical site, and an outer suction tube for extracting wound exudate, irrigation fluid, bone fragments, and debris from the surgical site.

The tip assembly 1 may be securely attached to the handpiece 11 of the pulse lavage device 2 and may be easily removed or replaced during or after use.

As used herein, the term "connector portion" means a component that acts as an interface between the tip assembly and the pulse lavage device. The connector portion may comprise internal conduits or channels to direct irrigation fluid, and suction forces, respectively.

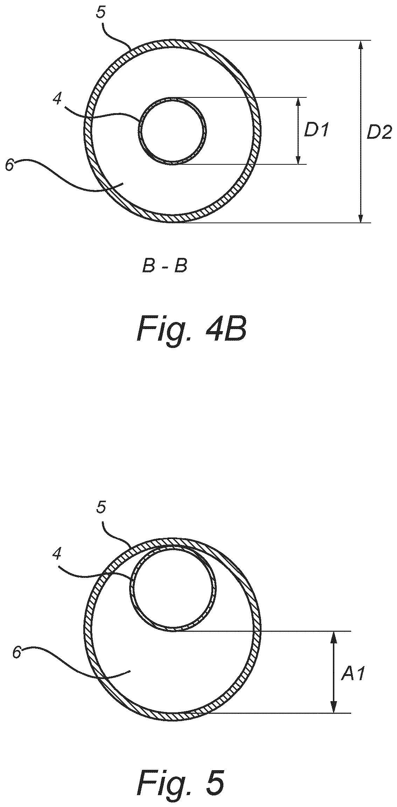

As best illustrated in figure 4B, and 5, the irrigation tube 4 is arranged within the suction tube 5 such that a suction channel 6 is defined between the irrigation tube 4 and the outer suction tube 5 in the mounted state.

The suction channel 6 allows for the removal of irrigation fluid, debris, and bone fragments from the surgical site.

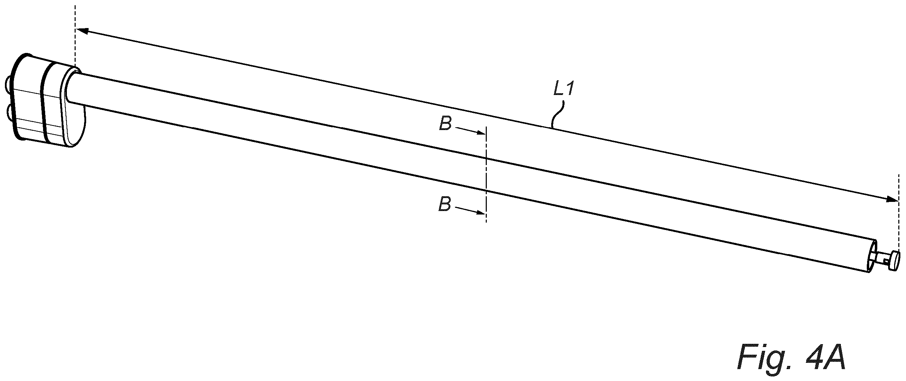

As best illustrated in figure 4A, the suction tube 5 is arranged to encircle at least 80% of the length, L₁, of the irrigation tube 4 in the mounted state. Preferably, the suction tube 5 may be arranged to encircle 80-99%, e.g. 90-98% of the length, L₁, of the irrigation tube 4 in the mounted state.

As used herein, the term "arranged to encircle" means that the suction tube is positioned around and fully surrounds the irrigation tube along a portion of its length, L₁.

The length, L₁, of the irrigation tube may be from 150 to 250 mm, e.g. from 160 to 200 mm.

The length, L₁, of the irrigation tube 4 is measured from a proximal end 4a to a distal end 4b of the irrigation tube 4.

The length of the suction tube may be from 2 to 15 mm smaller than the length, length, L₁, of the irrigation tube.

The length of the suction tube 5 is measured from a proximal end 5a to a distal end 5b of the suction tube 5.

The irrigation tube 4 has an outer surface and an inner surface. The suction tube 5 has an outer surface and an inner surface. The maximum distance, A₁, between the outer surface of the irrigation tube 4 and the inner surface of the suction tube 5 may be from 2 to 10 mm, preferably from 2 to 6 mm in the mounted state (see figure 5).

The irrigation tube 4 may be arranged centrally in the suction tube 5, as illustrated in figure 4B. Accordingly, a central axis of the irrigation tube 4 may coincide with a central axis of the suction tube 5. In such embodiments, the maximum distance, A₁, may also be referred to as the radial width. In figure 4B, the maximum distance, A₁, is not shown, but is represented by the distance (D₂ - D₁).

However, it is also conceivable that the irrigation tube 4 is arranged offset in relation to the suction tube 5, as illustrated in figure 5.

The irrigation tube 4 may be arranged in an off-center position within the suction tube 5 such that a central axis of the irrigation tube 4 is offset from a central axis of the suction tube 5 in the mounted state.

Accordingly, the central axes of the irrigation tube 4 and the suction tube 5 do not coincide.

In such embodiments, a portion of the inner surface of the suction tube 5 may be in direct contact with a portion of the outer surface of the irrigation tube 4 (see figure 5).

The irrigation tube 4 may have a diameter, D₁, of from 1.5 to 7 mm, preferably from 3 to 5 mm (see figure 4B).

The suction tube 5 may have a diameter, D₂, of from 3.5 to 15, preferably from 8 to 11 mm.

The irrigation tube 4 typically comprises a polymeric material. The polymeric material may be selected from the group consisting of polyurethane, polycarbonate, polyethylene, and/or silicone.

Typically, the irrigation tube 4 comprises a bendable polymeric material.

As used herein, the term "bendable polymeric material" refers to polymeric material that allows the irrigation tube to flex or deform without breaking or permanently deforming when subjected to an external force. An irrigation tube formed from a bendable polymeric material can conform to curved or confined anatomical spaces, such as bone cavities and intramedullary canals.

The irrigation tube is typically formed from a flexible and bendable polymeric material. This way, the irrigation tube may be inserted into confined anatomical spaces, such as bone cavities and intramedullary canals, and bend as needed.

Typically, the irrigation tube has a lower stiffness than the suction tube.
The suction tube preferably has a higher stiffness to secure that it remains stable and provides effective fluid removal without collapsing or deforming during use.

As used herein, the term "stiffness" refers to the resistance of a material or component to bending or deformation when subjected to an external force. In the context of the irrigation tube 4 and suction tube 5, stiffness may be quantified by the elastic modulus (Young's modulus) or flexural rigidity, which define the material's ability to maintain its shape under mechanical stress.

The suction tube 5 may be detachably attached to the connector portion 3 via a bayonet coupling, snap-fit mechanism, or threaded coupling.

A bayonet coupling is a "twist and lock" mechanism where opposing pins or projections on the suction tube may engage with corresponding slots on the connector portion. When the suction tube is inserted and rotated, the projections lock into place, providing a secure yet easily detachable connection.

A snap-fit mechanism is a flexible locking feature, which may be in the form of a ridge or protrusion on the suction tube, which may snap into a corresponding groove or recess on the connector portion.

A threaded coupling is a screw-type coupling or connection, where the suction tube may comprise threads that engage with threads on the connector portion. The threaded coupling may e.g. be a Luer lock fitting.

As best illustrated in figure 3A, the irrigation tube 4 has a proximal end 4a and a distal end 4b; the suction tube 5 having a proximal end 5a and a distal end 5b, wherein the proximal ends (4a, 5a) of the irrigation tube 4 and the suction tube 5 are attached to the connector portion 3 in the mounted state.

As best illustrated in figure 2A, the distal end 4b of the irrigation tube 4 may comprise one or more openings 7, wherein the one or more openings 7 are arranged in a direction radial to a central axis of the irrigation tube 4.

As used herein, the term "arranged in a direction radial to a central axis of the irrigation tube" means that the openings are positioned to direct fluid outward from the irrigation tube, perpendicular to its central axis.

This configuration is beneficial to secure that irrigation fluid is distributed outward from the tube, rather than being directed solely along its longitudinal axis. The radial fluid dispersion enhances bone surface cleaning and secures that irrigation fluid can reach surrounding areas. Accordingly, the debridement efficiency is improved.

In figure 2A, the one or more openings are provided in a separate tip 8, which is attached to the distal end 4b.

It is also conceivable that the one or more openings are provided in the irrigation tube 4.

The separate tip 8 may comprise a plurality of openings 7, wherein the openings 7 are circumferentially spaced around the separate tip 8 relative to the central axis of the irrigation tube 4.

For example, the separate tip 8 (or the irrigation tube 4) may comprise from 3 to 10 openings, e.g. from 3 to 5 openings.

The openings 7 are not limited to a specific shape, but may e.g. be round, oval, elliptical, or square-shaped. Each opening 7 may have a maximum width (e.g. a maximum diameter in case of round openings) of from 0.2 to 2 mm, e.g. from 0.5 to 1.5 mm.

That the openings are "circumferentially spaced around the separate tip relative to the central axis of the irrigation tube" means that the openings are positioned at regular intervals along the perimeter of the separate tip, i.e. along the perimeter of the irrigation tube.

This configuration is beneficial to secure a uniform lateral fluid distribution in all directions. Accordingly, the surgical site is evenly irrigated, which improves the debridement efficiency.

As best illustrated in figure 4A, the distal end 4b of the irrigation tube 4 may extend beyond the distal end 5b of the suction tube 5.

As mentioned hereinbefore, this configuration secures that irrigation fluid may be delivered directly to the surgical site without interference from the suction tube. It may also prevent premature suctioning.

Figure 3B illustrates that the suction tube 5 may be detachably attached to the connector portion 3 via a threaded coupling. As mentioned hereinbefore, the present disclosure is not limited to this mode of connection.

As shown in figure 3B, the connector portion 3 comprises an annular element 9 having an interior surface and an exterior surface, wherein the interior surface or the exterior surface is threaded; the proximal end 5a of the suction tube 5 may comprise threads 10 configured to engage with the threaded interior or exterior surface of the annular element 9.

In the embodiment illustrated in figure 3B, the exterior surface of the annular element 9 is threaded. Furthermore, the inner surface of the suction tube 5 is threaded, i.e. the inner surface comprises internal threads 10. It is, however, equally conceivable that the interior surface of the annular element 9 is threaded and that the outer surface of the suction tube 5 comprises external threads configured to engage with the threaded interior surface of the annular element 9.

A threaded coupling is beneficial as it allows for a secure and stable connection such that the suction tube remains firmly attached to the connector portion (or the annular portion thereof) during use. Furthermore, a threaded coupling facilitates the attachment and subsequent detachment of the suction tube, when needed. The threaded coupling is also suitable for repeated use.

The irrigation tube 4 may be fixedly or detachably attached to the connector portion 3. Typically, the irrigation tube 4 is fixedly attached to the annular element 9 of the connector portion 3.

This configuration may reduce the risk of accidental detachment of the irrigation tube, preventing fluid leakage. It may also simplify the assembly of the components of the tip assembly (and pulse lavage device). Furthermore, this configuration may be beneficial from a manufacturing aspect.

According to another aspect, there is provided a pulse lavage device 2 comprising the tip assembly 1 as described hereinbefore.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A tip assembly (1) for a pulse lavage device (2), wherein said tip assembly (1) comprises:
- a connector portion (3) for attaching said tip assembly (1) to said pulse lavage device (2);
- an inner irrigation tube (4) extending from said connector portion (3), wherein said irrigation tube (4) has a length, L₁; and
- an outer suction tube (5) configured to be detachably attached to said connector portion (3) and to encircle at least a portion of the length, L₁, of said irrigation tube (4) such that a suction channel (6) is defined between said irrigation tube (4) and said outer suction tube (5) in a mounted state.

2. The tip assembly (1) according to claim 1, wherein said suction tube (5) is configured to encircle at least 80%, preferably from 80 to 98% of the length, L₁, of said irrigation tube (4) in said mounted state.

3. The tip assembly (1) according to claim 1 or claim 2, wherein said irrigation tube (4) has an outer surface and an inner surface; said suction tube (5) having an outer surface and an inner surface, wherein the maximum distance, A₁, between said outer surface of said irrigation tube (4) and said inner surface of said suction tube (5) is from 2 to 10 mm, preferably from 2 to 6 mm in said mounted state.

4. The tip assembly (1) according to any one of claims 1-3, wherein said irrigation tube (4) is arranged in an off-center position within said suction tube (5) such that a central axis of said irrigation tube (4) is offset from a central axis of said suction tube (5) in said mounted state.

5. The tip assembly (1) according to any one of claims 1-4, wherein said irrigation tube (4) has a diameter, D₁, of from 1.5 to 7 mm, preferably from 3 to 5 mm.

6. The tip assembly (1) according to any one of claims 1-5, wherein said suction tube (5) has a diameter, D₂, of from 3.5 to 15 mm, preferably from 8 to 11 mm.

7. The tip assembly (1) according to any one of claims 1-7, wherein said irrigation tube (4) comprises a bendable polymeric material.

8. The tip assembly (1) according to claim 7, wherein said bendable polymeric material is selected from the group consisting of polyurethane, polycarbonate, polyethylene, and/or silicone.

9. The tip assembly (1) according to any one of claims 1-8, wherein said suction tube (5) is configured to be detachably attached to said connector portion (3) via a bayonet coupling, snap-fit mechanism, or threaded coupling.

10. The tip assembly (1) according to any one of claims 1-9, wherein said irrigation tube (4) has a proximal end (4a) and a distal end (4b); said suction tube (5) having a proximal end (5a) and a distal end (5b), wherein said proximal ends (4a, 5a) of said irrigation tube (4) and said suction tube (5) are attached to said connector portion (3) in said mounted state; said distal end (4b) of said irrigation tube (4) comprising one or more openings (7), wherein said one or more openings (7) is/are arranged in a direction radial to a central axis of said irrigation tube (4).

11. The tip assembly (1) according to claim 10, wherein said distal end (4b) comprises a separate tip (8) attached to said distal end (4b), and wherein said separate tip (8) comprises said one or more openings (7).

12. The tip assembly (1) according to claim 11, wherein said separate tip (8) comprises a plurality of openings (7), wherein said plurality of openings (7) is circumferentially spaced around said separate tip (8) relative to a central axis of said irrigation tube (4).

13. The tip assembly (1) according to any one of claims 10-12, wherein said distal end (4b) of said irrigation tube (4) extends beyond said distal end (5b) of said suction tube (5).

14. The tip assembly (1) according to any one of claims 10-13, wherein said connector portion (3) comprises an annular element (9) having an interior surface and an exterior surface, wherein said interior surface or said exterior surface is threaded; said proximal end (5a) of said suction tube (5) comprising threads (10) configured to engage with said threaded interior or exterior surface of said annular element (9).

15. A pulse lavage device (2) comprising the tip assembly (1) according to any one of claims 1-14.
